(19) Europäisches Patentamt
European Patent Office
Office européen des brevets

(11) Publication number: **0 074 259**
**B1**

(12) **EUROPEAN PATENT SPECIFICATION**

(45) Date of publication of patent specification: **17.07.85**

(51) Int. Cl.$^4$: **C 07 D 301/12**

(21) Application number: **82304656.0**

(22) Date of filing: **03.09.82**

# (54) Catalytic epoxidation of olefins.

(30) Priority: **04.09.81 JP 139502/81**
**26.11.81 JP 189709/81**

(43) Date of publication of application:
**16.03.83 Bulletin 83/11**

(45) Publication of the grant of the patent:
**17.07.85 Bulletin 85/29**

(84) Designated Contracting States:
**DE FR GB**

(56) References cited:
**BE-A- 848 521**
**FR-A- 739 562**
**US-A-3 806 467**

(73) Proprietor: **MITSUBISHI GAS CHEMICAL COMPANY, INC.**
**5-2, Marunouchi 2-chome Chiyoda-Ku**
**Tokyo (JP)**

(72) Inventor: **Kuriyama, Yasuhisa**
**No. 28-12, Kanamachi 3-chome**
**Katsushika-ku Tokyo (JP)**
Inventor: **Kakuda, Minoru**
**No. 14-6, Jinyamae Tokiwadaira**
**Matsudo-shi Chiba (JP)**
Inventor: **Nitoh, Shoichi**
**No. 14-16, Niijyuku**
**Katsushika-ku Tokyo (JP)**

(74) Representative: **Moore, Anthony John et al**
**Gee & Co. Chancery House Chancery Lane**
**London WC2A 1QU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 074 259 B1

**Description**

This invention relates to a process for producing olefin oxides by epoxidizing an olefin with hydrogen peroxide in the presence of an organotin compound as a catalyst to produce the corresponding olefin oxide.

Olefin oxides have been used for various purposes in the chemical industry. For example, olefin oxides have been used as intermediates for producing urethanes, glycol solvents, coating compositions, molding materials, surface active agents, plasticizers, and various other products, and the oxides have been produced by various processes.

Typical conventional processes for producing olefin oxides by epoxidation of olefins include the following three processes. The first process is a so-called "chlorohydrin process" and comprises reacting an olefin with chlorine or sodium hypochlorite in an alkaline medium to form chlorohydrin and subjecting the chlorohydrin so formed to dehydrochlorination to obtain an expoxide. The second process comprises air-oxidizing a hydrocarbon to form a hydroperoxide and epoxidizing an olefin with the hydroperoxide in the presence of a catalyst. This second process has the drawback that a large amount of alcohol is formed as a by-product from the hydroperoxide. The third process comprises air-oxidizing acetaldehyde to form peracetic acid, and epoxidizing an olefin with the peracetic acid. This third process also has a drawback in that a large amount of acetic acid formed as a by-product should be recovered.

In recent years, epoxidation of olefins by hydrogen peroxide has been proposed, and there are many reports on processes for producing olefin oxides which comprise reacting an olefin with hydrogen peroxide in an organic solvent in the presence of various types of epoxidation catalyst. The expoxidation catalysts used in these reported processes are mainly molybdenum and tungsten catalysts. These catalysts have a high catalytic activity, but they have the disadvantage that they easily decompose hydrogen peroxide. Boron and arsenic catalysts have also been reported as catalysts useful for providing epoxidation without causing such decomposition of hydrogen peroxide. However, the boron catalysts generally have a relatively low catalytic activity and the arsenic catalysts, despite their relatively high catalytic activity, have the disadvantage of high toxicity and, therefore, are not satisfactory for practical use in industry.

Furthermore, there have been proposed processes for epoxidation of olefins using a catalyst comprising an antimony compound and molybdenum or tungsten (Japanese Patent Publication No. 19216/80), or an organotin compound and molybdenum or tungsten (Japanese Patent Publications Nos. 25046/72 and 25323/72). Such catalysts have a relatively high catalytic activity, but are still unsatisfactory in their low selectivity and reaction efficiency with respect to hydrogen peroxide. We have found that catalysts comprising an organotin compound have both high activity and high selectivity.

Accordingly, the process of the present invention for producing an olefin oxide comprises reacting an olefin with hydrogen peroxide in the liquid phase in the presence of (a) a solvent comprising an organic carboxylic acid, wherein said organic carboxylic acid is present in an amount of 5% or more of volume based on the total volume of the liquid phase in the reaction system, and (b) a catalyst comprising at least one organotin compound represented by the general formula:

$$RSnXYZ \text{ or}$$

$$RSn(=O)X$$

wherein R represents an alkyl group having 1 to 12 carbon atoms, an aralkyl group having 7 to 12 carbon atoms or an aryl group having 6 to 12 carbon atoms; X, Y and Z, which may be the same or different, each represents a hydrogen or halogen atom, a hydroxyl group, an alkoxy group having 1 to 12 carbon atoms, an acyloxy group having 1 to 12 carbon atoms or an aryl group having 6 to 12 carbon atoms.

Olefins to be epoxidized by the process of this invention are olefinically unsaturated hydrocarbon compounds and include substituted or unsubstituted straight chain or cyclic olefins. Generally, these olefins contain from 2 to 20 carbon atoms and at least one double bond. Such olefins may be represented by the following general formulae:

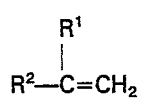

wherein $R^1$ and $R^2$, which may be the same or different, each represents hydrogen or a substituted or unsubstituted straight chain or branched chain alkyl group having from 1 to 20 carbon atoms;

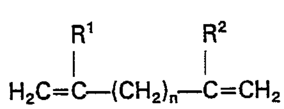

wherein $R^1$ and $R^2$ are as defined above, and $n$ is 0 or an integer of from 1 to 10;

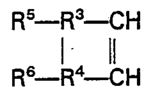

wherein $R^3$ and $R^4$, which may be the same or different, each represents an alkylene group having from 1 to 4 carbon atoms, and $R^5$ and $R^6$, which may be the same or different, each represents hydrogen or a substituted or unsubstituted straight chain or branched chain alkyl group having from 1 to 10 carbon atoms. These cyclic olefins thus have up to 10 carbon atoms and an unsaturated bond in the cyclic moiety thereon and may be substituted by one or two alkyl groups having from 1 to 10 carbon atoms; and,

$$R^7R^9C{=}CR^8R^{10}$$

wherein $R^7$ and $R^8$, which may be the same or different, each represents a straight chain or branched chain alkyl group having from 1 to 10 carbon atoms and $R^9$ and $R^{10}$ each represents hydrogen or the same group as that of $R^7$ and $R^8$; and

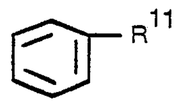 or 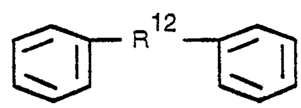

wherein $R^{11}$ represents a monovalent alkenyl group having from 2 to 10 carbon atoms, and $R^{12}$ represents a divalent alkenyl group having from 2 to 5 carbon atoms.

Typical examples of olefins represented by the above formulae include aliphatic olefins such as propylene, butene, isobutene or hexene; cycloolefins such as cyclopentene, cyclohexene or cyclooctene; alkyl- or alkenylcycloolefins such as methylcyclohexene, methylcyclopentene or vinylcyclohexene; alkenyl aromatic hydrocarbons such as styrene, vinylbenzene or methylstyrene; conjugated or non-conjugated diolefins such as 1,5-cyclooctadiene, 1,5,9-cyclodecatriene, 1,4-cyclohexadiene or butadiene; olefinic alcohols such as allyl alcohol, methylvinylcarbinol or cyclohexanol; halogenated olefins such as allyl chloride or allyl bromide; and unsaturated aliphatic acids and esters thereof such as acrylic acid, methacrylic acid, crotonic acid, oleic acid, allyl acetate, soybean oil or linseed oil.

The organotin compound used as a catalyst in the present invention should not have a substantial content of other metals such as molybdenum, tungsten or vanadium.

The catalyst is of the formula RSnXYZ or RSn(=0)X, as defined above.

Examples of the above organotin compounds are $(C_2H_5)_4Sn$, $(C_6H_5)_4Sn$, $CH_3SnCl_3$, $C_6H_5SnCl_3$, $(C_4H_9)_2SnCl_2$, $(C_6H_5)_2SnCl_2$, $(C_4H_9)_3SnCl$, $(C_6H_5)_3SnCl$,

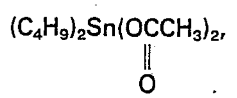, 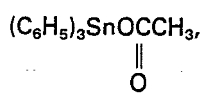,

$(C_6H_5)_3SnH$, $C_4H_9Sn(O)OH$, $(C_6H_5)_3SnOC_2H_5$, $(C_4H_9)_2SnO$, $(C_6H_5)_3SnSn(C_6H_5)_3$, $(C_6H_5)_2Sn$, $(C_6H_5)_3Sn{-}O{-}Sn(C_6H_5)_3$, $(CH_3)_3Sn{-}N(C_2H_5)_2$, $(C_4H_9)_3Sn{-}O{-}Si(C_4H_9)_3$ and $[(C_2H_5)_3Sn]_2S$.

Of these organotin compounds, preferred compounds are those represented by the formula RSn(=0)X, and particularly preferred compounds are organic stannonic acids represented by the formula RSn(=0)OH. Specific examples of these organotin compounds are methylstannonic acid

ethylstannonic acid 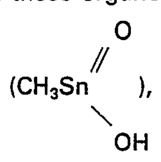, butylstannonic acid 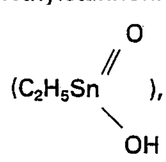, octylstannonic acid 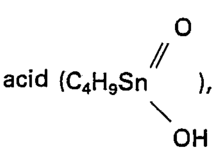,

phenylstannonic acid 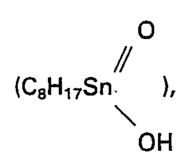,

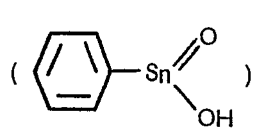, methylphenylstannonic acid

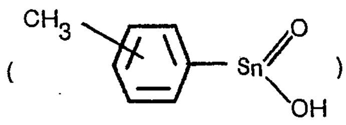
, methoxyphenylstannonic acid

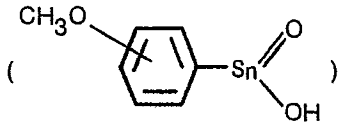
, carboxyphenylstannonic acid

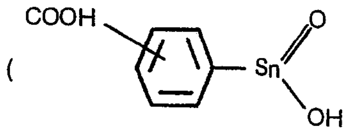
, chlorophenylstannonic acid

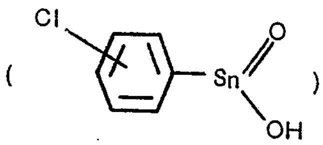
and nitrophenylstannonic acid

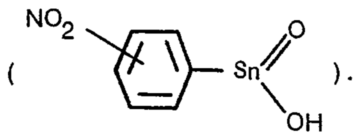
.

It is preferred that the organotin compounds used as the catalysts in the present invention are soluble or partially soluble in the reaction system; however, sparingly soluble or insoluble compounds can be used as well. Furthermore, conventional techniques for achieving sufficient contact of the reactants with the catalyst may be used in the present invention; for example, the catalyst may be supported on an inert carrier such as silica or alumina.

The reaction is carried out in a solvent which comprises at least 5%, by volume of the liquid phase, of an organic carboxylic acid; this solvent system must provide a liquid reaction system and be inactive to the reactants and catalyst. A solvent compound other than the carboxylic acid can be selected from alcohols, esters, ethers, saturated hydrocarbons, halogenated hydrocarbons, aromatic hydrocarbons and organic carboxylic acids. Examples of the solvents include *n*-propanol, *iso*-propanol, *n*-butanol, *iso*-butanol, *t*-butanol, amyl alcohol, cyclohexanol, ethylene glycol, propylene glycol, ethyl ether, isopropyl ether, dioxane, tetrahydrofuran, ethylene oxide, methyl acetate, ethyl acetate, phenyl acetate, cyclohexyl acetate, *n*-pentane, cyclohexane, cyclohexene, dichloromethane, dichloroethane, dichloropropane, tetrachloroethane, benzene, toluene, xylene, chlorobenzene, dichlorobenzene, acetic acid, propionic acid, butyric acid, isobutyric acid and benzoic acid.

Examples of these organic carboxylic acid solvents or solvent systems are acetic acid, propionic acid, butyric acid, isobutyric acid, acetic acid-benzene, acetic acid-dichloromethane, acetic acid-dichloroethane, acetic acid-dichloropropane, propionic acid-benzene, propionic acid-cyclohexane, pripionic acid-dichloromethane, propionic acid-dichloroethane, propionic acid-dichloropropane, propionic acid-*t*-butanol, propionic acid methyl acetate, propionic acid-dioxane and *m*-chlorobenzoic acid-dioxane. With respect to the solvent, the type of solvent used (e.g., whether the organic acid is used alone or in combination with other solvents) or the ratio of the solvents can be suitably determined by taking into consideration such factors as the type of olefin being used in the reaction, the type of epoxide to be produced and whether ease of operation or economy is more important. For example, if the epoxide produced is easily ring-opened by an organic carboxylic acid, the ring opening reaction can be avoided or minimized by reducing the proportion of the organic carboxylic acid in the solvent.

In the process of the invention, it is preferred to use the hydrogen peroxide at a concentration such that the reactants other than the catalyst are maintained in as uniform a phase as possible under the reaction conditions. Accordingly, a hydrogen peroxide aqueous solution having a concentration of from 30 to 90% (by weight) is generally used. However, in some instances, hydrogen peroxide having higher concentrations may be required because some olefins easily cause ring opening by moisture present in the reaction system. In such a case, it is preferred to perform the reaction while removing water from the reaction system by azeotropic distillation with the reaction solvent or by continuously introducing an inert gas such as nitrogen, argon or helium into the reactor; or to use a process in which water is removed by adding a dehydrating agent such as anhydrous sodium sulfate or anhydrous magnesium sulphate to the reaction system.

The reaction temperature may be selected over a wide margin depending upon the activity or the catalyst used, the reactivity of the olefin used, the ease of ring-opening of the formed epoxy compounds

and the type of solvent selected, but it is generally in the range of from 0° to 150°C, preferably from 40 to 120°C. The reaction time is generally from 5 to 240 minutes. Furthermore, the reaction can be carried out under atmospheric pressure or a pressurized condition so long as the reaction system can be maintained in a liquid phase. Generally a pressure from 1 to 150 atm. is preferably used.

The concentration of the catalyst used in the present invention may be selected fairly widely, and the concentration at which the catalyst exhibits a preferred catalytic activity can be suitably selected according to the type of the antimony compound or the organotin compound used as the catalyst, the type and the reactivity of olefin used, etc. Accordingly, the catalyst is used in an amount required for providing catalytic activity, which is generally 1/2000 to 1/2 mol, preferably, 1/400 to 1/5 mol per mol of hydrogen peroxide. Furthermore, the concentration of olefin in the reaction system is not critical, but it can be generally used in a molar ratio (olefin to hydrogen peroxide) of 1:30 to 30:1.

In addition, it is desirable to add a stabilizer, for example, a chelating agent such as ethylenediaminetetraacetic acid, diethylenetriaminepentaacetic acid, cyclohexanediaminetetraacetic acid, aminotrimethylenephosphonic acid, 1-hydroxyethylidene-1,1-diphosphonic acid or ethylenediaminetetramethylenephosphonic acid, because the hydrogen peroxide reactant is relatively unstable and easily decomposed during the reaction by contamination with, for example, heavy metals, thereby reducing the yield of the desired olefin oxides.

As described above, the process of the present invention is a remarkably advantageous for practising on an industrial scale because the epoxidation of olefins with hydrogen peroxide can be carried out in a high yield and a high selectivity by using as a catalyst one or more of the antimony or organotin compounds defined above.

The present invention is illustrated in greater detail by the following Examples in which the percentages are by weight.

## Example 1

A four-necked flask having a capacity of 100 cc and equipped with a water separator and a reflux condenser was charged with 15 ml of *t*-butanol and 5 ml of propionic acid as a solvent. Then, 50 ml of cyclohexene were added thereto and the mixture was refluxed. 0.103 mol of 90% hydrogen peroxide was then added dropwise thereto over 10 minutes with stirring. After completion of the addition, the mixture was allowed to react under reflux for 30 minutes while removing water formed during the reaction by the water separator. After completion of the reaction, the amount of unreacted hydrogen peroxide was found to be 0.006 mol and, upon analysis by gas chromatography, the cyclohexene oxide produced was found to be 0.084 mol. These results indicate that cyclohexene oxide was produced in a conversion of 94% and a selectivity of 87%, based on the amount of hydrogen peroxide used.

## Example 2

A reaction was carried out in the same manner as described in Example 1 for 120 minutes using 20 ml of *t*-butanol. In this reaction, cyclohexene oxide was produced in a conversion of 47% and a selectivity of 40%, based on the amount of hydrogen peroxide used.

## Example 3

A 100 cc four-necked flask was charged with 45 ml of propionic acid as a solvent and 0.5 g of phenylstannonic acid as a catalyst. Then, 20 ml of allyl chloride were added thereto and the mixture was refluxed. 0.101 mol of 90% hydrogen peroxide was then added dropwise over 10 minutes with stirring. After completion of the addition, the mixture was allowed to react for 110 minutes under reflux. After completion of the reaction, the amount unreacted hydrogen peroxide was found to be 0.018 mol and, upon analysis by gas chromatography, the epichlorohydrin produced was found to be 0.077 mol. These results indicate that epichlorohydrin was produced in a conversion of 82% and a selectivity of 93%, based on the amount of hydrogen peroxide used.

## Example 4

A reaction was conducted in the same manner as described in Example 3 but using 0.5 g of diphenyltin oxide as a catalyst. In this reaction, epichlorohydrin was produced in a conversion of 80% and a selectivity of 94%, based on the amount of hydrogen peroxide used.

## Example 5

A 200 cc glass autoclave was charged with 20 ml of propionic acid and 20 ml of dichloroethane as a solvent system and 0.5 g of octylstannonic acid as a catalyst. Then, 20 ml of allyl chloride, 0.02 g of ethylenediaminetetramethylenephosphonic acid as a stabilizer and 0.101 mol of 90% hydrogen peroxide were added thereto and the mixture was allowed to react at 100°C for 60 seconds with stirring. After completion of the reaction, the amount of unreacted hydrogen peroxide in the reaction solution was found to be 0.010 mol and, upon analysis of the products in the reaction solution by gas chromatography, the epichlorohydrin produced was found to be 0.082 mol. These results indicate that epichlorohydrin was produced in a conversion of 90% and a selectivity of 90%, based on the amount of hydrogen peroxide used.

Example 6

A stainless (SUS 316) autoclave having a capacity of 300 cc was charged with 25 ml of dichloromethane, 10 ml of *t*-butanol and 5 ml of propionic acid as a solvent system and 0.5 g of butyl stannonic acid as a catalyst. Then, 0.102 mol of 90% hydrogen peroxide and 0.40 mol of propylene were added thereto and the mixture was allowed to react at 70°C for 60 minutes. After completion of the reaction, the amount of unreacted hydrogen peroxide was found to be 0.029 mol and, upon analysis by gas chromatography, the propylene oxide produced was found to be 0.063 mol. These results indicate that propylene oxide was produced in a conversion of 72% and a selectivity of 86%, based on the amount of hydrogen peroxide used.

**Claims**

1. A process for producing an olefin oxide, which comprises reacting an olefin with hydrogen peroxide in the liquid phase in the presence of (a) a solvent comprising an organic carboxylic acid, wherein said organic carboxylic acid is present in an amount of 5% or more by volume based on the total volume of the liquid phase in the reaction system, and (b) a catalyst comprising at least one organotin compound represented by the general formula:

$$RSnXYZ \text{ or}$$
$$RSn(=O)X$$

wherein R represents an alkyl group having 1 to 12 carbon atoms, an aralkyl group having 7 to 12 carbon atoms or an aryl group having 6 to 12 carbon atoms; X, Y and Z, which may be the same or different, each represents a hydrogen or halogen atom, a hydroxyl group, an alkoxy group having 1 to 12 carbon atoms, an acyloxy group having 1 to 12 carbon atoms or an aryl group having 6 to 12 carbon atoms.

2. A process according to Claim 1, wherein said organotin compound is represented by the general formula:

$$RSn(=O)X$$

wherein R and X are as defined above.

3. A process according to Claim 2, wherein said organotin compound is represented by the formula:

$$RSn(=O)OH$$

wherein R is as defined above.

4. A process according to Claim 3, wherein said organotin compound is methylstannonic acid, ethylstannonic acid, butylstannonic acid, octylstannonic acid, phenylstannonic acid, methylphenylstannonic acid, methoxyphenylstannonic acid, carboxyphenylstannonic acid, chlorophenylstannonic acid or nitrophenylstannonic acid.

5. A process according to any preceding claim, wherein the catalyst is used in an amount of from 1/2000 to 1/2 mol per mol of hydrogen peroxide.

6. A process according to any preceding claim, wherein the molar ratio of said olefin to hydrogen peroxide is from 1:30 to 30:1.

**Patentansprüche**

1. Verfahren zur Herstellung eines Olefinoxids, bei dem man ein Olefin mit Wasserstoffperoxid in der flüssigen Phase in Gegenwart von (a) einem Lösungsmittel, umfassend eine organische Carbonsäure, wobei die organische Carbonsäure in einer Menge von 5 % oder mehr, bezogen auf das Gesamtvolumen der flüssigen Phase im Reaktionssystem, vorliegt, und (b) einem Katalysator, umfassend wenigstens eine Organozinnverbindung, dargestellt durch die allgemeine Formel

$$RSnXYZ \text{ oder}$$
$$RSn(=O)X$$

worin R eine Alkylgruppe mit 1 bis 12 Kohlenstoffatomen, eine Aralkylgruppe mit 7 bis 12 Kohlenstoffatomen oder eine Arylgruppe mit 6 bis 12 Kohlenstoffatomen bedeutet; X, Y und Z, die gleich oder verschieden sein können, jeweils ein Wasserstoff- oder Halogenatom, eine Hydroxylgruppe, eine Alkoxygruppe mit 1 bis 12 Kohlenstoffatomen, eine Acyloxygruppe mit 1 bis 12 Kohlenstoffatomen oder eine Arylgruppe mit 6 bis 12 Kohlenstoffatomen bedeuten, umsetzt.

2. Verfahren gemäss Anspruch 1, worin die Organozinnverbindung die allgemeine Formel

$$RSn(=O)X$$

hat, worin R und X die vorher angegebenen Bedeutungen haben.

3. Verfahren gemäss Anspruch 2, worin die Organozinnverbindung die allgemeine Formel

RSn(=0)OH

hat, worin R die vorher angegebene Bedeutung hat.

4. Verfahren gemäss Anspruch 3, worin die Organozinnverbindung Methylzinnsäure, Ethylzinnsäure, Butylzinnsäure, Octylzinnsäure, Phenylzinnsäure, Methylphenylzinnsäre, Methoxyphenylzinnsäure, Carboxyphenylzinnsäure, Chlorophenylzinnsäure oder Nitrophenylzinnsäure ist.

5. Verfahren gemäss einem der vorhergehenden Ansprüche, worin der Katalysator in einer Menge von 1/2000 bis 1/2 Mol pro Mol Wasserstoffperoxid verwendet wird.

6. Verfahren gemäss einem der vorhergehenden Ansprüche, worin das Molarverhältnis von Olefin zu Wasserstoffperoxid 1:30 bis 30:1 beträgt.

**Revendications**

1. Un procédé pour la production d'un oxyde d'oléfine, qui comprend la réaction d'une oléfine avec le peroxyde d'hydrogène en phase liquide en présence (a) d'un solvant comprenant un acide carboxylique organique, ledit acide carboxylique organique étant présent en quantité de 5 % ou plus en volume, par rapport au volume total de la phase liquide dans le système de réaction, et (b) d'un catalyseur comprenant au moins un composé d'organo-étain représenté par la formule générale:

RSnXYZ, ou
RSn(=0)X

où R représente un groupe alkyle en $C_1$—$C_{12}$, un groupe arylalkyle en $C_7$—$C_{12}$ ou un groupe aryle en $C_6$—$C_{12}$, X, Y et Z, qui peuvent être identiques ou différents, représentent chacun un atome d'hydrogène ou d'halogène ou un groupe hydroxyle, un groupe alcoxy en $C_1$—$C_{12}$, un groupe acyloxy en $C_1$—$C_{12}$ ou un groupe aryle en $C_6$—$C_{12}$.

2. Un procédé selon la revendication 1, dans lequel ledit composé d'organo-étain est représenté par la formule générale:

RSn(=0)X

dans laquelle R et X sont comme défini ci-dessus.

3. Un procédé selon la revendication 2, dans lequel ledit composé d'organo-étain est représenté par la formule générale:

RSn(=0)OH

dans laquelle R est comme défini ci-dessus.

4. Un procédé selon la revendication 3, dans lequel ledit composé d'organo-étain est l'acide méthylstannonique, l'acide éthylstannonique, l'acide butylstannonique, l'acide octylstannonique, l'acide phénylstannonique, l'acide méthylphénylstannonique, l'acide méthoxyphénylstannonique, l'acide carboxyphénylstannonique, l'acide chlorophénylstannonique ou l'acide nitrophénylstannonique.

5. Un procédé selon l'une quelconque des revendications précédentes, dans lequel le catalyseur est utilisé en quantité de 1/2 000 à 1/2 mol par mol de peroxyde d'hydrogène.

6. Un procédé selon l'une quelconque des revendications précédentes, dans lequel le rapport molaire de ladite oléfine au peroxyde d'hyrogène est de 1:30 à 30:1.